# EUROPÄISCHE PATENTSCHRIFT

(11) **EP 2 358 400 B1**
(45) Veröffentlichungstag und Bekanntmachung des Hinweises auf die Patenterteilung: **03.09.2014**
(21) Anmeldenummer: 09796311.0
(22) Anmeldetag: 16.12.2009
(51) Int. Cl.: A61L 15/26, A61L 15/42, A61F 2/78, A61L 27/34, C08G 61/02

(54) **ORTHOPÄDISCHES POLSTER UND VERFAHREN ZU SEINER HERSTELLUNG**
ORTHOPAEDIC CUSHION AND METHOD FOR PRODUCTION THEREOF
COUSSIN ORTHOPÉDIQUE ET SON PROCÉDÉ DE FABRICATION

(30) Priorität: 19.12.2008 DE 102008063818
(43) Veröffentlichungstag der Anmeldung: 24.08.2011
(73) Patentinhaber: Otto Bock HealthCare GmbH, 37115 Duderstadt (DE)
(72) Erfinder: ANHALT, Klaus-Peter, 37434 Rhumspringe (DE)
(74) Vertreter: Lins, Martina
(86) Internationale Anmeldenummer: PCT/EP2009/009012
(87) Internationale Veröffentlichungsnummer: WO 2010/078924

(56) Entgegenhaltungen:
- WO-A1-03/051241
- DE-U1- 20 315 575
- US-B1- 6 270 872

## Beschreibung

Die Erfindung betrifft ein Verfahren zur Herstellung eines reibvermindernd beschichteten orthopädischen Polsters aus einem gefüllten Elastomer, einem Elastomergel, einem weichgemachten Thermoplasten oder einem Polyurethan, ein neues so erhältliches orthopädisches Polster und die Verwendung des Beschichtungsmaterials Poly(para-Xylylen) auf einem orthopädischen Polster..

Orthopädische Polster werden unter anderem für die Abpolsterung zwischen orthopädischen Hilfsmitteln und Körperteilen, beispielsweise für die Abpolsterung zwischen Protheseteilen, Ortheseteilen oder Gestellteilen und dem Körper des Benutzers eingesetzt. Zu den orthopädischen Polstern zählen wir hier insbesondere sogenannte Liner und Schäfte.

Unter einem orthopädischen Liner versteht man eine polsternde oder formende Hülse für das distale Ende eines Gliedmaßenstumpfes oder einer Gliedmaße. Allgemein versteht man unter einem Liner auch jedes polsternde und formende Material in Form eines Strumpfes, einer Hülle, einer Binde oder eines Patches für den polsternden Übergang zwischen einem Körperteil und einem orthopädischen Mittel, z.B. einem Prothesenschaft, einem Stützapparat oder einer Orthese.

Insbesondere die Stumpfliner müssen ähnlich wie Stützstrümpfe eng sitzen und werden daher für das Anziehen umgeschlagen und auf den Stumpf aufgerollt. Das Linermaterial wird dabei mechanisch stark beansprucht und muss hoch-elastisch und biegsam sein. Durch das Aufziehen auf den Stumpf werden die Liner hohen Zugkräften ausgesetzt. Für den Patienten kann die Prozedur sehr belastend und anstrengen sein. Es werden daher Artziehilfen und Anziehsprays angeboten, wobei letztere gesundheitlich bedenklich sein können.

Um die Reibungswerte auf der Liner-Außenseite zu verringern, werden bei bekannten Linern zum Teil die Gleitfähigkeit erhöhende (reibungsmindemde) Beschichtungen vorgesehen. Die Wirkungsweise einer gleitfähigen Beschichtung beruht jedoch häufig auf einer Glättung der Oberfläche, so dass die bekannten Beschichtungen in relativ dicker Schicht aufgetragen werden müssen. Die Beschichtung kann dann die für die Funktion gewünschten Materialeigenschaften des Liners beeinträchtigen. Dies gilt sinngemäß auch für andere Polster.

Aus der WO 03/051241 A1 ist ein Elastomerliner bekannt, der mit Poly(para-Xylylen) beschichtet ist. Diese Beschichtung wird durch chemische Dampfphasenabscheidung (CVD) aufgebracht und zeichnet sich durch ausgezeichnete Gleitfähigkeit aus. Sie verbindet sich gut mit Silikon und nicht gefüllten Synthesekautschuken. Die CVD-Technik ermöglicht einen dünneren Auftrag, der die Materialeigenschaften des Liners weniger beeinträchtigt und sparsam mit dem Beschichtungsmaterial umgeht.

Die DE 203 15 575 U1 offenbart einen Liner oder eine Kniekappe aus einem elastischen Material wie Silikon oder Polyurethan dessen Außenseite durch eine Beschichtung mit einem polymeren Material gleitfähig ausgerüstet ist. Die Beschichtung kann beispielsweise durch sprühen, tauchen, aufrakeln oder dergleichen aufgebracht werden, insbesondere nachdem eine Haftvermittlerschicht durch Plasma- oder Coronabehandlung oder durch Beflämmen des Linermaterials aufgebracht wurde.

Die US 6,270,872 B1 offenbart ein orthopädisches Poster, das mit Parylene beschichtet ist und das zusätzlich äußerlich mit einem Klebstoff beschichtet ist, um an einem menschlichen Körper haften zu können. Das Polsterbasismaterial ist ein Elastomer, vorzugweise ein Polysiloxanpolymer. Die Parylenebeschichtung wird mit CVD aufgebracht.

Die Beschichtung aus Polyparaxylylen ist eine in den verschiedensten Anwendungsgebieten, unter anderem auf elektronischen Bauteilen und in der Medizintechnik verwendete die Gleitfähigkeit erhöhende Beschichtung, die lösungsmittelfest und chemisch inert ist. Polyparaxylylene sind Polymere einer zweibindigen Xylol-Einheit des Aufbaus -CH₂-(C₆H₅₋ₙ)Rₙ-CH₂-, mit n = 1 bis 3 und verschiedenen möglichen Substituenten R, unter anderem Chlor, Fluor und Amin. Diese Polymere werden heute in einem Standard-CVD-verfahren aus den pyrolysierten Dimeren auf den verschiedensten Oberflächen und unter anderem auf orthopädischen Polstern wie z. B. Linern abgeschieden.

Außerdem sind für Liner andere gleitfähige Beschichtungen aus Polytetrafluorethylen und sonstigen Trockengleitfilmen bekannt.

Wie sich nun herausgestellt hat, können zwar Liner aus Silikon gut mit Hilfe von CVD-Verfahren mit Trockengleitfilmen beschichtet werden, die Beschichtung anderer Kunststoff-Polstermaterialien ist jedoch schwierig, besonders, wenn diese fluide gefüllt sind. Die Beschichtung von gefüllten Elastomeren, Elastomergelen, insbesondere Polyurethan-Gelen, oder weichgemachten Thermoplasten (Weichkunststoffen, wie z. B. Weich-PVC) und ebenso von hydrophilem Polyurethan allgemein ist mit bekannten CVD- oder PVD-Beschichtungsverfahren nicht möglich, bzw. gelingt nicht in der gewünschten Qualität.

Besonders die Beschichtung von weichen Polyurethanen und Polyurethan-Gelen mit Trockengleitfilmen ist bislang nicht dünnschichtig möglich. Polyurethanliner, insbesondere Polyurethan-Gel-Liner und weiche Polyurethanliner, werden daher häufig nur mit Sprays und flüssigen oder weichen Schmiermitteln behandelt. Eine Beschichtung mit TPU (thermoplastischem Polyurethan) aus Lösung ist qualitativ nicht befriedigend. Bei Beschichtung von Polyurethanlinern mit nicht-polyurethan-basierten, unpolaren bzw. nicht hydrophilen Polymeren ergeben sich ungleichmäßige Abscheidungen, schwankende Schichtdicken, Flecken, Risse und großflächige Ablösungen. Die so beschichteten Liner sind unbrauchbar und zeigen, soweit überhaupt verwendbar, vorzeitige Abnutzung bzw. hohen Verschleiß.

Entsprechendes gilt für Beschichtungen, die auf anderen orthopädischen Polstern aufgebracht werden sollen.

Der Erfindung liegt die Aufgabe zugrunde, ein orthopädisches Polster aus einem fluid gefüllten Elastomer, einem Elastomergel, einem weich gemachten Thermoplasten oder einem Polyurethan mit einer reibungsvermindernden, die Gleitfähigkeit der Oberflächen auf sich selbst und der Haut eines Benutzers erhöhenden Beschichtung zu versehen, die sich gleichmäßig fest haftend auftragen lässt, die funktionellen Materialeigenschaften des Polsters nicht beeinträchtigt und bei starker mechanischer Belastung des Polsters, insbesondere bei Dehnung des Polsters haltbar ist.

Diese Aufgabe wird verfahrensmäßig durch die Merkmale des Anspruchs 1 gelöst.

Die unpolare, die Gleitfähigkeit erhöhende Polymerbeschichtung ist eine solche, die die Gleitfähigkeit der Polster-oberflächen auf einander, gegenüber orthopädischen Teilen oder Textilien oder sich selbst erhöht.

Überraschender Weise wurde gefunden, dass sich Wasser und andere flüchtige Bestandteile, wie in dem Material vorhandene Hilfs- und Zusatzstoffe, Lösemittel und Verarbeitungshilfsmittel oder Monomerreste, in dem gezielten, unmittelbar vor die Beschichtung geschalteten Vorbehandlungsschritt aus der Oberfläche entziehen lassen, woraufhin eine gleichmäßig haftende Beschichtung aufgetragen werden kann, ohne dass dem Polstermaterial über das Gesamtvolumen zu viel seiner für die mechanische Brauchbarkeit wesentlichen Weichmacher und/oder Extender (fluide Füller) entzogen würden.

Durch die Vorbehandlung wird zunächst offenbar eine Nichtgleichgewichtsverteilung erzeugt, die eine Beschichtung der Oberfläche in guter Qualität ermöglicht.

Wenn das Polstermaterial aus weichmacherfreiem Polyurethan besteht, dient die Vorbehandlung in erster Linie dazu, von dem hydrophilen Polyurethan aus der Umgebung aufgenommenes Wasser zu entfernen, da der Wassergehalt andernfalls eine gleichmäßige Beschichtung verhindert.

Die Erfindung kann auf verschiedensten gefüllten oder weichgemachten Materialien oder allgemein auf Polyurethanen angewendet werden.

Im Falle, dass Polyurethan für das erfindungsgemäße Polster eingesetzt wird, handelt es sich dabei besonders bevorzugt um ein weiches Polyurethan oder ein Polyurethan-Gel. Als "weich" werden hier solche Polyurethane bezeichnet, deren Shore-00 Härte zwischen 15 und 50, vorzugsweise zwischen 20 und 30 liegt; sie erfordern dabei keinen Weichmacher. Weiche Polyurethane und Polyurethan-Gele sind für orthopädische Zwecke besonders geeignet, sie sind in der Regel hydrophil.

Gele sind allgemein Systeme aus einem makromolekularen oder kolloidalen Netzwerk (feindisperse und gerüstbildende feste Phase), das mit einem fluiden freier beweglichen Medium gefüllt ist (Expander-Phase). Wenn die Expander-Phase Wasser ist spricht man von einem Hydrogel. Der Begriff Polyurethan-Gele umfasst Polyurethan-Hydrogele und hier vorzugsweise reine Polyurethan-Gele, bei denen sowohl die hochmolekulare Gerüstphase als auch die fluide Expanderphase aus Polyurethankomponenten bestehen. Dabei kann insbesondere ein locker vernetztes oder auch untervernetztes Polyurethangerüst mit überschüssiger isocyanatreaktiver Komponente (im Allgemeinen Polyol) gefüllt sein.

Für die erfindungsgemäßen Polster lassen sich auch andere Elastomergele oder gefüllten Elastomere einsetzen. "Gefüllt" sind Elastomere dann, wenn eine bei Raumtemperatur oder Körpertemperatur flüssige Substanz enthalten ist, die nicht chemisch kovalent in die Vernetzung des Elastomers eingebunden ist. Bei einem lockeren Vernetzungsgrad und einem hohen Füllungsgrad kann man das Füllungsmittel als Gel-Dispersionsmittel und das gefüllte Elastomer insgesamt auch als ein Elastomer-Gel ansehen. Wenn der Füller Wasser ist, bezeichnet man die zugehörigen Gele als "Hydrogele". Als Ölfüller werden insbesondere Silikonöle oder Kohlenwasserstofföle wie z. B. medizinische Weißöle verwendet.

Beispiele für gefüllte Elastomere sind insbesondere ölgefüllte Kautschukpolymere und besonders ölgefüllte Kautschuk-Copolymere. Die Kautschuke umfassen Naturkautschuk und Synthesekautschuke.

Für die Polstermaterialien geeignete Elastomere, einschließlich thermoplastischer Elastomere, sind insbesondere Copolymere mit α-Olefinen und/oder Styrol, insbesondere Styrol-Butadien-Styrol-Block-Copolymer (SBS), Styrol-Ethylen-Butylen-Styrol-Block-Copolymer (SEBS), Styrol-Isopren-Styrol-Copolymer (SIS), Styrol,-Ethylen-Propylen-Copolymere (SEP), (SEEPS), (SEPS), Styrol-Ethylen-Butylen-Copolymer (SEB)

Weiterhin sind als Polstermaterialien weich gemachte Thermoplaste geieignet, insbesondere Weich-PVC. Diese enthalten Weichmacher um die ansonsten spröden und harten Polyvinylchloride für Polster geeignet zu machen.

Vakuumbehandlung und Trocknung werden alternativ oder in Kombination durchgeführt, und zwar vorzugsweise jeweils einzeln oder gemeinsam für einen experimentell in Vorversuchen zu bestimmenden Zeitraum. Polyurethanpolster können bis zur Gewichtskonstanz behandelt werden. Andere Vorbehandlungsschritte können vor, nach oder gegebenenfalls (falls getrennt vorgenommen) zwischen dem Vakuumbehandlungs- und dem Trocknungsschritt durchgeführt werden.

"Unmittelbar anschließend" bedeutet, dass keine längeren Lager- oder Transportzeiten zwischen Vorbehandlung und Beschichtungsschritt zwischengeschaltet werden dürfen, d.h. dass das Verfahren zwischen Vorbehandlung und Beschichtung nicht unterbrochen werden darf, da das Material andernfalls flüchtige Stoffe aus dem Inneren des Polsters an die Oberfläche nachliefern kann, dabei aber versprödet, oder im Falle von Polyurethan erneut Wasser aufnehmen könnte. Derzeit wird davon ausgegangen, dass zwischen Vorbehandlung und Beschichtung nicht mehr als 12 Stunden vergehen dürfen sofern das vorbehandelte Polster nicht durch besondere Maßnahmen während der Lagerung geschützt wird. Eine Lagerung im Vakuum oder in einer Trockenkammer oder ein Tempern des Polsters bei erhöhter Temperatur unter ausgewählten Bedingungen, z.B. in bestimmter Gasatmosphäre, zählen ggf. zur Vorbehandlung und sind unter Umständen, z. B. bei Polyurethan, möglich.

Für das Entziehen von Wasser und anderen flüchtigen Bestandteilen kann als alleinige Maßnahme eine Trocknung durchgeführt werden, die möglichst schonend, d.h. bei moderater Temperatur erfolgen sollte. Die maximal verwendbare Temperatur richtet sich nach den Daten des verwendeten Polstermaterials. Vorzugsweise wird bei einer Temperatur zwischen Raumtemperatur und 80 °C gearbeitet. In einer bevorzugten Ausführungsform wird das Polster in einem Ofen bei einer Temperatur zwischen 30 und 60 °C, zum Beispiel bei etwa 40 °C behandelt. Polyurethanpolster können über Nacht bis zur Gewichtskonstanz getrocknet werden. Die Trocknung kann jedoch auch mit anderen Maßnahmen zum Entziehen flüchtiger Bestandteile, insbesondere einer Vakuumbehandlung, kombiniert werden.

Alternativ zur Trocknung bei erhöhter Temperatur in einem Ofen kann für das Entziehen von Wasser und anderen flüchtigen Bestandteilen eine Behandlung im Vakuum durchgeführt werden, vorzugsweise über einen Zeitraum zwischen einer halben und mehreren Stunden, weiter vorzugsweise über 2 bis 8 Stunden, insbesondere über 4 bis 6 Stunden oder bis zur Gewichtskonstanz. Geeignet ist eine Behandlung bei einem Restdruck von ca. 0,1 bis 100 mbar, vorzugsweise 5 bis 15 mbar.

Die Behandlung kann in alternativer Ausführungsform in einer Vakuumtrockenkammer unter gleichzeitig erhöhter Temperatur (beispielsweise 25 bis 40 °C) erfolgen.

Erfindungsgemäß erfolgt die Beschichtung mit Hilfe eines chemischen oder physikalischen Gasphasenabscheidungsverfahrens. Diese Verfahren sind Stand der Technik bekannt und müssen daher nicht näher beschrieben werden. Von einer chemischen Gasphasenabscheidung (CVD) spricht man dann, wenn das Material der Beschichtung sich aus Prozessgasen, die die Ausgangsstoffe beinhalten, während des Bedampfens im Gasraum und/oder auf der zu beschichtenden Oberfläche bildet. Von einer physikalischen Gasphasenabscheidung (PVD) spricht man dann, wenn ein Beschichtungsmaterial ohne chemische Veränderung lediglich verdampft und wieder abgeschieden wird. Geeignete Apparaturen zur Durchführung dieser Verfahren sind beispielsweise aus US 3,246,627, US 3,301,707 oder US 3,600,216 bekannt.

Als eine "dünnen" Beschichtung kann eine solche angesehen werden, deren Schichtdicke bis zu 10 µm, vorzugsweise bis zu 5 µm und weiter vorzugsweise nur bis zu 2,5 µm beträgt. Wesentlich für die Qualität der Beschichtung ist auch, dass die Schichtdicke gleichmäßig ist.

Der Vorbehandlungsschritt für das Entziehen von Wasser und anderen flüchtigen Bestandteilen vor dem Beschichten kann in der Beschichtungskammer der CVD- oder PVD-Apparatur selbst erfolgen, und zwar mit oder ohne Temperaturerhöhung. Hierfür wird vor dem Einleiten des Gasgemisches für die Beschichtung für eine bestimmte Zeit ein Vakuum (ohne Prozessgaseinleitung) angelegt.

Vorzugsweise ist jedoch vorgesehen, dass das zu beschichtende Polster vor der Beschichtung in der Behandlungskammer des Beschichtungsapparats in einer gesonderten, vorgeschalteten Vakuumkammer für etwa eine halbe bis sechs Stunden einem Vakuum ausgesetzt wird, wie es auch für die Beschichtung vorgesehenen ist. Anschließend wird das Polster in die CVD-Kammer überführt und beschichtet.

Vor dem Beschichten kann als zusätzlicher oder alternativer Vorbehandlungsschritt eine Plasmabehandlung oder Coronabehandlung der zu beschichtenden Oberfläche erfolgen. Dies ist in besonders vorteilhafter Weise in der Beschichtungskammer einer CVD- oder PVD-Apparatur möglich, die häufig für diese Vorbehandlung bereits ausgerüstet sind.

In besonders bevorzugter Ausführungsform kann als Beschichtungsmittel ein Poly(para-Xylylen) verwendet werden, insbesondere ein nichtsubstituiertes oder halogensubstituiertes Polyparaxylylen. Das Mittel kann unter dem Handelsnamen Parylene ® (Parea Tech Coating Inc, USA) bezogen werden. Alternativ kommen andere die Gleitfähigkeit des Polstermaterials erhöhende Polymerbeschichtungen, insbesondere sogenannte Trockengfeüfilme, in Frage. Zu den Trockengleitfilmen zählen auch Tetrafluorethylen und dessen verschiedene Abkömmlinge.

Zusätzlich kann es von Vorteil sein, dass das Polster innerhalb der Vorbehandlung wenigstens einem zusätzlichen Reinigungsschritt unterworfen wird. Dies erfolgt vorzugsweise vor dem Trocknen und/oder Vakuumbehandeln des Materials, kann jedoch auch noch zwischen- oder nachgeschaltet werden. Der Reinigungsschritt kann aus einem Waschen mit Wasser oder Wasser und Seife bestehen, oder aus einem Waschen oder Spülen mit einem anderen Mittel, z.B. einem organischen Lösungsmittel, vorzugsweise dann einem Alkohol. Der Reinigungsschritt kann auch ein Tränken oder Quellen lassen umfassen. Bevorzugt ist beispielsweise ein anfängliches Waschen des auspolymerisierten Polsters mit Wasser und Seife, nur mit Wasser, mit Isopropanol oder mit Ethanol. Mehrere Reinigungsschritte sind kombinierbar, wobei sie direkt aufeinander folgen oder an verschiedenen Stellen des Vorbehandlungsverfahrens eingeschoben werden können.

Eine Ausführungsform der Erfindung kann sein, dass zunächst ein Waschen und Trocknen durchgeführt wird, dann ein Spülen mit organischem Lösungsmittel und danach ein weiterer Trocknungs- und/oder Vakuumbehandlungsschritt.

Weiterhin ist es gemäß einer Ausführungsform der Erfindung vorgesehen, dass vor dem Beschichten ein Haftvermittler aufgebracht wird. Geeignet sind bekannte silanbasierte Haftvermittler, wie sie beispielsweise in der Kautschukindustrie verwendet werden. Die Verwendung eines Haftvermittlers wird jedoch nicht als grundsätzlich erforderlich angesehen, insbesondere nicht, wenn vor dem Beschichten eine Plasma- oder Coronabehandlung der Oberfläche vorgenommen wird.

Die Erfindung stellt folglich erstmals ein orthopädisches Polster aus einem gefüllten Elastomer, einem Elastomergel, einem weich gemachten Thermoplasten oder einem Polyurethan mit einer dünnen reibungsvermindernden unpolaren und nicht hydrophilen Polymerbeschichtung in einer gleichmäßigen, dünnen Schichtdicke zur Verfügung. Derartig beschichtete Polster, insbesondere Liner oder Schäfte, wie in Anspruch 8 gekennzeichnet, waren vorher nicht herstellbar.

Grundsätzlich können alle weichen und gefüllten Materialien mit dem erfindungsgemäßen Verfahren beschichtet werden, Bei dem erfindungsgemäßen orthopädischen Poster handelt es sich jedoch vorzugsweise um einen Liner, einen Schaft insgesamt oder ein gesondertes Schaftpolster. Zu den bevorzugten. Polstern, bei denen die Erfindung angewendet wird, zählen auch Orthesepolster, die auf diese Weise staubund schmutzabweisend gemacht werden sollen. Weiterhin können polsternde Bandagen aller Art beschichtet werden, z.B. Fuß-, Knie- oder Ellenbogenbandagen.

Die Polymerbeschichtung kann sich vorzugsweise sowohl auf der Außenseite des Polsters als auch der in Anwendungsposition der Haut zugewandten Innenseite des Polsters befinden. Aufgrund seiner hohen Anschmiegsamkeit an den Körper des Benutzers rutscht auch ein Polyurethan-Gelpolster trotz reibungsvermindernder innen liegender Beschichtung nicht. Zusätzlich zur äußeren Beschichtung des Polsters, die beispielsweise die Außenfläche eines Liners leichter auf sich selbst gleiten lässt und so das An- und Ausziehen des Liners erleichtert, kann das Polster nach dieser Erfindung eine Innenbeschichtung aufweisen, die das völlig rutschfeste Verhalten vieler Polstermaterialien aufhebt und damit ebenfalls wesentlich zum Tragekomfort beiträgt.

In besonders bevorzugter Ausführungsform besteht die Polymerbeschichtung aus einem Poly(para-Xylylen), wie oben bereits angegeben.

Besonders bevorzugt im Sinne der Erfindung ist es auch, wenn die Beschichtung gerade in solcher Schichtdicke aufgetragen wird, dass sie bei Dehnung des Liners um 40 % in wenigstens eine Raumrichtung nicht reißt, vorzugsweise bei einer Dehnung um 50 % und weiter vorzugsweise bei einer Dehnung µm 100% nicht reißt. Es wurde gefunden, dass die Beschichtung umso elastischer und reißfester ist, je dünner sie aufgetragen wird. Dem steht entgegen, dass eine größere Schichtdicke zu einer längeren praktischen Brauchbarkeit des Liners beiträgt, da sie einen unvermeidlichen Abrieb ausgleicht. Als optimal werden daher Schichtdicken zwischen 50 und 500 nm oder 50 und 1000 nm angesehen, die einer Dehnung des Liners in wenigstens eine Längenrichtung von bis zu 100 % standhalten können ohne zu-reißen, oder wenn eine etwas längere Gebrauchsdauer gewünscht wird, Schichtdicken über 500 nm bis 2,5 µm, die Dehnungen bis mindestens 40 % standhalten können.

Die optimale Schichtdicke hängt daher sehr stark vom Polstermaterial und der Form, Art und Anwendungsposition des Polsters ab, und ist vom Fachmann nach Bedarf auszuwählen und zu erproben.

In vorteilhafter Weiterbildung kann sich zwischen Polstermaterial und Polymerbeschichtung ein Haftvermittler befinden, wie oben bereits beschrieben. Geeignete Haftvermittler sind dem Fachmann bekannt, insbesondere können gängige Silan-Haftvermittler eingesetzt werden. Das Polstermaterial ist wie oben zum Verfahren schon angegeben.

Im Weiteren werden einige beispielhafte Ausführungsmöglichkeiten für die Erfindung angegeben:
Es wurden Liner aus Polyurethangelen angefertigt Die Polyurethangele hierfür wurden hergestellt aus:
   A: Glyzerin gestartetem Polyetherpolyol auf der Basis von Propylenoxid mit 10 bis 20 % endständigem Ethylenoxid und mittleren Molekulargewichten von ca. 5000 bis 6000 und B: modifiziertem HDI mit einem NCO-Gehalt von 10 bis 20 %; im Verhältnis A:B zwischen 100:5 und 100:30.

Als Zusatzstoffe wurden lediglich inerte anorganische Füllstoffe und PU-Katalysator verwendet.

### I. Vorbehandlungsbeispiele:

### Verfahrensbeispiel 1

Die PU-Liner Oberfläche wird zunächst mit Wasser und Seife gereinigt, anschließend im Ofen bei 40 °C über Nacht bis zur Gewichtskonstanz getrocknet. Danach wird eine Beschichtung durchgeführt.

### Verfahrensbeispiel 2

Die PU-Liner Oberfläche wird mit Isopropanol gereinigt, anschließend im Vakuum bei 10 bis 20 mbar Restdruck über 4 bis 6 Stunden bis zur Gewichtskonstanz entgast. Danach wird eine Beschichtung durchgeführt.

### Verfahrensbeispiel 3

Die PU-Liner Oberfläche wird wie unter 2 beschrieben vorbehandelt. Unmittelbar vor der Beschichtung wird im Vakuum in der gleichen Behandlungskammer ein Plasma gezündet um die Oberfläche in situ vorzubehandeln.

### Verfahrensbeispiel 4

Die PU-Liner Oberfläche wird für
- 5 bis 10 min in Isopropanol getränkt;
- 5 bis 10 min mit destilliertem Wasser gespült;
- 30 min in einer Mischung aus 100 Teilen Isopropanol und 1 Teil Silan A 174 (GE-Silicones) behandelt;
- getrocknet
- 5 min mit Isopropanol gespült;
- 30 min bei 115 °C temperiert;
- nach Verfahrensbeispiel 1 oder 2 weiter vorbehandelt.

Danach wird eine Beschichtung durchgeführt.

### II. Beschichtungsbeispiel

Für eine CVD-Beschichtung mit Poly(para-Xylylen) - unmodifiziert - wird das cyclische Di-Paraxylylen im Vakuum bei einer Temperatur von ca. 150 °C verdampft und dann bei Temperaturen bis zu 650 °C in einer Pyrolysekammer pyrolysiert, um freie Paraxylyl-Monomere im Gaszustand zu erzeugen. Das so erzeugte Monomer-haltige Prozessgas wird in eine Vakuumkammer eingeführt, die den zu beschichtenden Liner enthält. Der Liner wird in der Prozess-Vakuumkammer so gehaltert, dass die zu beschichtenden Oberflächen exponiert, d.h. mit dem Monomer-haltigen Gas in Berührung gebracht werden. Die Prozess-Vakuumkammer mit dem Liner kann bei annähernd Raumtemperatur, d.h. Temperaturen zwischen 20 und 30 °C gehalten werden. Das Polymer bzw. das in situ polymerisierende Monomer schlägt sich auf den exponierten Liner-Oberflächen nieder. Die Dicke des entstehenden Beschichtungsfilms kann u. a. über die Dauer des Beschichtungsvorgangs eingestellt werden.

Im Folgenden werden ein erfindungsgemäßes und ein Vergleichsbeispiel in Bezug auf die Gebrauchseigenschaften verglichen.

### Vergleichsbeispiel

Ein auspolymerisierter PU-Liner der oben angegebenen Spezifikation wurde mit Wasser und Seife und danach mit Isopropanol gewaschen, um ihn von Trennmittel, Fett und anhaftendem Schmutz zu befreien, und dann dem unter B. beschriebenen Beschichtungsverfahren unterzogen. Die Ergebnisse sind in Tabelle 1 angegeben.

### erfindungsgemäßes Beispiel

Ein auspolymerisierter PU-Liner der oben angegebenen Spezifikation wurde gemäß einem Versuchsbeispiel vorbehandelt und dann dem unter B. beschriebenen Beschichtungsverfahren unterworfen. Die Ergebnisse sind in Tabelle 1 zusammengefasst. Alle Liner nach den Versuchsbeispielen 1 bis 4 erfüllten die Spezifikationen laut rechter Tabellenspalte.

**Tabelle 1**

| Eigenschaft | Vergleichsbeispiel | erfindungsgemäßes Beispiel |
|---|---|---|
| Aussehen | fleckig, ungleichmäßig, farblich schimmernd (weil in unterschiedlichen Bereichen unterschiedlich starke Schichtabscheidung zu Interferenzerscheinungen des Lichtes führt) | gleichmäßig milchiges, einheitliches Aussehen ohne Farbnuancen |
| Einreißverhalten | Beschichtung reißt aufgrund der sehr stark unterschiedlichen Schichtabscheidungen an den Stellen mit sehr geringer und an den Stellen mit sehr dicker Schicht schnell ein | gleichmäßige Schicht führt zu einem gleichmäßigen Reißen erst bei verhältnismäßig hoher Dehnung |
| Dehnungsverhalten | Kräuseln der Oberfläche beim Dehnen | unverändertes Erscheinungsbild beim Dehnen und Entlasten |
| Gleiteigenschaften | starker Stick-Slip-Effekt beim aufeinander Abgleiten der Schichten | glatte, gleitfähige Beschichtung, welche beim aufeinander Abgleiten zu keinem Stick-Slip-Effekt führt |
| Reibungsminderung (nach ASTM 1894) Angabe als einheitenloser Koeffizient (Force to move the sample horizontal)/(Downward force of sled), je kleiner der Koeffizient, umso geringer ist die Reibung | statisch 1,5 - 2,6 | statisch 0,3 - 0,6 |
| | kinetisch 1,9 - 2,9 | kinetisch 0,2 - 0,5 |

## Patentansprüche

1. Verfahren zur Herstellung eines reibvermindernd beschichteten orthopädischen Polsters aus einem gefüllten Elastomer, einem Elastomergel, einem weichgemachten Thermoplasten oder einem Polyurethan, **dadurch gekennzeichnet, dass** das auspolymerisierte Polster wenigstens einem Vorbehandlungsschritt in Form einer Vakuum- und/oder Trocknungsbehandlung unterworfen wird, in welcher flüchtige Bestandteile, nämlich in dem Polstermaterial vorhandene Hilfs- und Zusatzstoffe, Lösemittel, Verarbeitungshilfsstoffe, Monomere und/oder Wasser, aus der Oberfläche entzogen werden, und dass das Polster unmittelbar anschließend mit einer unpolaren, die Gleitfähigkeit erhöhenden Polymerbeschichtung beschichtet wird, wobei die Beschichtung mit Hilfe eines chemischen oder physikalischen Gasabscheidungsverfahrens erfolgt.

2. Verfahren nach Anspruch 1, **dadurch gekennzeichnet, dass** für das Entziehen von flüchtigen Bestandteilen eine Trocknung in einem Ofen bei einer Temperatur zwischen 30 °C und 60 °C durchgeführt wird.

3. Verfahren nach Anspruch 1 oder 2, **dadurch gekennzeichnet, dass** für das Entziehen von flüchtigen Bestandteilen eine Behandlung im Vakuum bei einem Restdruck von 0,1 bis 100 mbar durchgeführt wird.

4. Verfahren nach einem der Ansprüche 1 bis 3, **dadurch gekennzeichnet, dass** das auspolymerisierte und vorbehandelte Polster mit einem polymeren Trockengleitfilm beschichtet wird.

5. Verfahren nach einem der Ansprüche 1 bis 4, **dadurch gekennzeichnet, dass** vor dem Beschichten eine Plasmabehandlung oder Coronabehandlung der zu beschichtenden Polsteroberfläche erfolgt.

6. Verfahren nach einem der Ansprüche 1 bis 5, **dadurch gekennzeichnet, dass** als Beschichtungsmittel Poly(para-Xylylen) verwendet wird.

7. Verfahren nach einem der Ansprüche 1 bis 6, **dadurch gekennzeichnet, dass** vor dem Beschichten ein Haftvermittler aufgebracht wird.

8. Orthopädisches Polster aus einem gefüllten Elastomer, einem Elastomergel, einem weichgemachten Thermoplasten oder einem Polyurethan mit einer dünnen reibungsvermindernden Beschichtung, erhältlich mit einem Verfahren nach einem der Ansprüche 1 bis 7, **dadurch gekennzeichnet, dass** die Beschichtung eine unpolare, die Gleitfähigkeit erhöhende Polymerbeschichtung in einer gleichmäßigen Schichtdicke von bis zu 10 µm ist und dass die Beschichtung gerade in solcher Schichtdicke aufgetragen wird, dass sie bei Dehnung des Liners um 40 % in wenigstens eine Raumrichtung nicht reißt und vorzugsweise bei einer Dehnung um 100 % nicht reißt.

9. Orthopädisches Polster nach Anspruch 8, **dadurch gekennzeichnet, dass** es sich um einen Liner, einen Schaft, ein Schaftpolster, ein Orthesepolster oder eine polsternde Bandage handelt.

10. Orthopädisches Polster nach Anspruch 8 oder 9, **dadurch gekennzeichnet, dass** die Polymerbeschichtung sich sowohl auf der Außenseite als auch der in Anwendungsposition hautzugewandten Innenseite befindet.

11. Orthopädisches Polster nach einem der Ansprüche 8 bis 10, **dadurch gekennzeichnet, dass** die Polymerbeschichtung aus einem Poly(paraXylylen) besteht.

12. Orthopädisches Polster nach einem der Ansprüche 8 bis 11, **dadurch gekennzeichnet, dass** die Beschichtung eine Schichtdicke von bis zu 2,5 µm besitzt.

13. Orthopädisches Polster nach einem der Ansprüche 8 bis 12, **dadurch gekennzeichnet, dass** sich zwischen Polstermaterial und Polymerbeschichtung ein Haftvermittler befindet.

## Claims

1. Process for producing an orthopaedic cushion which has a friction-reducing coating and which is made of a filled elastomer, of an elastomer gel, of a plasticized thermoplastic, or of a polyurethane, **characterized in that** the fully polymerized cushion is subjected to at least one pretreatment step in the form of a vacuum treatment and/or drying treatment, where volatile constituents, namely water, monomers, processing aids, solvents and/or additives and auxiliaries present in the cushion material, are extracted out of the surface, and **in that** the cushion is subsequently immediately coated with a nonpolar polymer coating which increases slip capability, the coating process using a chemical or physical gas-phase deposition process.

2. Process according to Claim 1, **characterized in that**, for the extraction of volatile constituents, a drying process is carried out in an oven at a temperature of from 30°C to 60°C.

3. Process according to Claim 1 or 2, **characterized in that**, for the extraction of volatile constituents, a treatment in vacuo at a residual pressure of from 0.1 to 100 mbar is carried out.

4. Process according to any of Claims 1 to 3, **characterized in that** the fully polymerized and pretreated cushion is coated with a polymeric dry friction-reducing film.

5. Process according to any of Claims 1 to 4, **characterized in that**, prior to the coating process, plasma treatment or corona treatment can take place on the cushion surface to be coated.

6. Process according to any of Claims 1 to 5, **characterized in that** poly(para-xylylene) is used as coating agent.

7. Process according to any of Claims 1 to 6, **characterized in that**, prior to the coating process, an adhesion promoter is applied.

8. Orthopaedic cushion made of a filled elastomer, of an elastomer gel, of a plasticized thermoplastic, or of a polyurethane, with a thin friction-reducing coating, obtainable by a process according to any of Claims 1 to 7, **characterized in that** the coating is a uniform layer thickness of up to 10 µm of a nonpolar polymer coating which increases slip capability and **in that** the coating is applied specifically in a layer thickness such that it does not split on extension of the liner by 40% in at least one spatial direction, and preferably does not split on extension by 100%.

9. Orthopaedic cushion according to Claim 8, **characterized in that** it involves a liner, a socket, a socket cushion, an orthotic cushion, or a cushioning bandage.

10. Orthopaedic cushion according to Claim 8 or 9, **characterized in that** the location of the polymer coating is not only on the external side but also on the internal side which, in usage position, faces toward the skin.

11. Orthopaedic cushion according to any of Claims 8 to 10, **characterized in that** the polymer coating is composed of a poly(para-xylylene).

12. Orthopaedic cushion according to any of Claims 8 to 11, **characterized in that** the layer thickness of the coating is up 2.5 µm.

13. Orthopaedic cushion according to any of Claims 8 to 12, **characterized in that** there is an adhesion promoter located between cushion material and polymer coating.

## Revendications

1. Procédé pour la réalisation d'un coussin orthopédique revêtu réduisant la friction à partir d'un élastomère chargé, d'un gel élastomère, d'une matière thermoplastique rendue souple ou d'un polyuréthane,
**caractérisé en ce que** le coussin entièrement polymérisé est soumis à au moins une étape de traitement sous la forme d'un traitement sous vide et/ou d'un traitement de séchage dans lequel les composants volatiles sont extraits hors de la surface, à savoir les agents auxiliaires et additifs présents dans le matériau de coussin, les solvants, les produits auxiliaires de traitement, les monomères et/ou l'eau, et **en ce que** le coussin est revêtu immédiatement ensuite avec un revêtement polymère monopolaire, augmentant la capacité de glissement, et le revêtement a lieu avec l'aide d'un procédé de déposition chimique ou physique en phase gazeuse.

2. Procédé selon la revendication 1, **caractérisé en ce que** pour la suppression des composants volatiles on exécute un séchage dans un four à une température entre 30° C et 60° C

3. Procédé selon la revendication 1 ou 2, **caractérisé en ce que** pour la suppression des composants volatiles on exécute un traitement sous vide avec une pression résiduelle de 0,1 à 100 mbar.

4. Procédé selon l'une des revendications 1 à 3, **caractérisé en ce que** le coussin entièrement polymérisé et prétraité est revêtu d'un film de glissement à sec en polymère.

5. Procédé selon l'une des revendications 1 à 4, **caractérisé en ce qu'**avant le revêtement on procède à un traitement au plasma ou un traitement Corona de la surface du coussin à revêtir.

6. Procédé selon l'une des revendications 1 à 5, **caractérisé en ce que** l'on utilise du poly(para-xylylène) à titre d'agent de revêtement.

7. Procédé selon l'une des revendications 1 à 6, **caractérisé en ce que** l'on applique un intermédiaire d'adhésion avant le revêtement.

8. Coussin orthopédique réalisé en élastomère chargé, en gel d'élastomère, en matière thermoplastique rendue souple ou en polyuréthane avec un revêtement mince réduisant la friction, susceptible d'être obtenu avec un procédé selon l'une des revendications 1 à 7, **caractérisé en ce que** le revêtement est un revêtement de polymère monopolaire augmentant la capacité de glissement avec une épaisseur de couche régulière allant jusqu'à 10 µm, et **en ce que** le revêtement est appliqué exactement avec des épaisseurs de couche telles que pour un allongement du revêtement de 40 % dans au moins une direction dans l'espace, il ne se déchire pas et de préférence pour un allongement d'environ 100 % il ne se déchire pas.

9. Coussin orthopédique selon la revendication 8, **caractérisé en ce qu'**il s'agit d'un doublage, d'une tige, d'un coussin de tige, d'un coussin d'orthèse ou d'un bandage à effet coussin.

10. Coussin orthopédique selon la revendication 8 ou 9, **caractérisé en ce que** le revêtement de polymère se trouve aussi bien sur la face extérieure que sur la face intérieure tournée vers la peau dans la position d'utilisation.

11. Coussin orthopédique selon l'une des revendications 8 à 10, **caractérisé en ce que** le revêtement de polymère est en poly(para-xylylène).

12. Coussin orthopédique selon l'une des revendications 8 à 11, **caractérisé en ce que** le revêtement possède une épaisseur de couche allant jusqu'à 2,5 µm.

13. Coussin orthopédique selon l'une des revendications 8 à 12, **caractérisé en ce qu'**un intermédiaire d'adhésion se trouve entre le matériau de coussin et le revêtement de polymère.
